# EUROPÄISCHE PATENTSCHRIFT

(11) **EP 2 029 186 B1**
(45) Veröffentlichungstag und Bekanntmachung des Hinweises auf die Patenterteilung: **01.12.2010**
(21) Anmeldenummer: 07725658.4
(22) Anmeldetag: 30.05.2007
(51) Int. Cl.: A61L 27/24, C08L 89/06, C12N 5/00

(54) **ISOLIERTES NATURIDENTISCHES KOLLAGEN**
ISOLATED NATURE-IDENTICAL COLLAGEN
COLLAGÈNE ISOLÉ IDENTIQUE AU NATUREL

(30) Priorität: 31.05.2006 DE 102006026591
(43) Veröffentlichungstag der Anmeldung: 04.03.2009
(73) Patentinhaber: Fraunhofer-Gesellschaft zur Förderung der angewandten Forschung e.V., 80686 München (DE)
(72) Erfinder: XIONG, Xin, 70569 Stuttgart (DE); MERTSCHING, Heike, 71063 Sindelfingen (DE); RUPP, Steffen, 70569 Stuttgart (DE); BRUNNER, Herwig, 82362 Weilheim (DE)
(86) Internationale Anmeldenummer: PCT/EP2007/004767
(87) Internationale Veröffentlichungsnummer: WO 2007/137827

(56) Entgegenhaltungen:
- DE-A1- 10 062 623
- STOLTZ M ET AL: "NONHELICAL REGIONS IN RAT COLLAGEN ALPHA 1 CHAIN" FEBS LETTERS, Bd. 26, Nr. 1, 1972, Seiten 61-65, XP002450468 ISSN: 0014-5793
- BECKER U ET AL: "AMINO TERMINAL EXTENSIONS ON SKIN COLLAGEN FROM SHEEP WITH A GENETIC DEFECT IN CONVERSION OF PRO COLLAGEN INTO COLLAGEN" BIOCHEMISTRY, Bd. 15, Nr. 13, 1976, Seiten 2853-2862, XP002450469 ISSN: 0006-2960
- BECKER U ET AL: "CARBOXY TERMINAL ANTIGENIC DETERMINANTS OF COLLAGEN FROM CALF SKIN LOCALIZATION WITHIN DISCRETE REGIONS OF THE NONHELICAL SEQUENCE" EUROPEAN JOURNAL OF BIOCHEMISTRY, Bd. 28, Nr. 2, 1972, Seiten 221-231, XP002450470 ISSN: 0014-2956
- ADELMANN B C: "THE STRUCTURAL BASIS OF CELL MEDIATED IMMUNOLOGICAL REACTIONS OF COLLAGEN REACTIVITY OF SEPARATED ALPHA CHAINS OF CALF AND RAT COLLAGEN IN CUTANEOUS DELAYED HYPER SENSITIVITY REACTIONS" IMMUNOLOGY, Bd. 23, Nr. 5, 1972, Seiten 739-748, XP002450471 ISSN: 0019-2805

## Beschreibung

Die vorliegende Erfindung betrifft isoliertes Kollagen, Verfahren zur Herstellung und Isolierung des Kollagens aus kollagenhaltigem Gewebe, sowie die Verwendung des isolierten Kollagens in einer Biomatrix, als in vitro-Testsystem, Gewebeersatz oder Organersatz.

### Stand der Technik

Extrazelluläre Matrices (ECM) oder Trägerstrukturen für die Kultivierung von Zellen, sogenannte Biomatrices, werden meist aus isoliertem Kollagen, das heißt aus kollagenhaltigem Gewebe isolierten Matrixproteinen hergestellt. Vor allem in der regenerativen Medizin spielen solche Biomatrices eine wichtige Rolle. Dabei hat das sogenannte Tissue Engineering zum Ziel, erkrankte, ausgefallene oder verloren gegangene Gewebe- oder Organfunktionen zu unterstützen oder zu ersetzen. Dazu werden in der Regel ex vivo Gewebe- oder Organstrukturen gezüchtet, indem auf bereitgestellten Trägerstrukturen oder Biomatrices mindestens ein gewebespezifischer Zelltyp ausgesät und kultiviert wird. Biomatrix und darauf kultivierte Zellen bilden den Gewebe- oder Organäquivalent oder -ersatz.

Für die Realisierung gewebespezifischer Funktionen ist es notwendig, dass die auf die Biomatrix aufgebrachten Zellen die Matrixproteine reorganisieren beziehungsweise diese neu bilden. Für eine erfolgreiche Kultivierung der Zellen auf solchen Trägerstrukturen ist es deshalb wichtig, dass Matrixproteine vorhanden sind, die möglichst native oder naturidentische Eigenschaften aufweisen.

Bekannte Biomatrices werden meist aus Kollagen hergestellt, welches aus frisch präpariertem kollagenhaltigem Gewebe, beispielsweise Haut oder Sehnen extrahiert, das heißt isoliert wird. ,

Ein bekanntes Extraktionsverfahren ist die Extraktion mit wässriger Essigsäure. In üblichen Extraktionsprotokollen wird Essigsäure in einer Konzentration von 0,1 % bis zu 500 mmol/l eingesetzt. Zur Extraktion von Kollagen aus kollagenhaltigem Gewebe, beispielsweise Rattenschwanzsehnen (RTT), wird dieses mit der Essigsäure über einen Zeitraum von 20 Stunden bis 7 Tagen meist bei Raumtemperatur gerührt. Nachteilig ist, dass aufgrund der hohen Viskosität der Kollagenlösung die Endkonzentration an Kollagen in der Extraktfraktion begrenzt ist. Nachteilig ist vor allem, dass die native Struktur der extrahierten Matrixproteine während der Inkubation aufgrund saurer Hydrolyse durch die Essigsäure und aufgrund enzymatischen Verdaus durch meist vorhandene Proteinasen angegriffen und die Matrixproteine dabei teilweise oder vollständig denaturiert werden. Die anschließende Regenerierung der ursprünglichen nativen Kollagenstruktur und die Wiederherstellung der biologischen Funktionen der Matrixproteine sind deshalb begrenzt. Werden die mittels Essigsäureextraktion isolierten und teilweise denaturierten Matrixproteine anschließend noch lyophilisiert, weichen die Eigenschaften des unter Verwendung des Lyophilisats wiederhergestellten Kollagenprodukts stark von nativem Kollagen ab; dass dessen Anwendung zur Kultivierung von gewebespezifischen Zellen weitgehend eingeschränkt ist. Darüberhinaus weisen die aus den mittels Essigsäureextraktion isolierten Matrixproteinen hergestellten Biomatrices weitere Nachteile wie unkontrollierte (meist eindimensionale, vertikale) Schrumpfung auf.

Die Verwendung solcher bekanntermaßen hergestellter Biomatrices für in vitro Testsysteme, beispielsweise zur Testung neuer Wirkstoffe, ist stark eingeschränkt, weil die kultivierten Zellen aufgrund der vorhandenen denaturierten Matrixproteine oft gegenüber einem in vivo- oder in situ-Zustand nachteilig veränderte physiologische und morphologische Eigenschaften und zelluläre Funktionen entwickeln. Die Übertragbarkeit der in vitro Testergebnisse wird so erschwert.

Es besteht der Bedarf an alternativen und verbesserten Mitteln und Verfahren zur einfachen Gewinnung von verbessertem, das heißt weitgehend naturidentischem, isoliertem Kollagen und daraus hergestellten Biomatrices, die die Nachteile des Standes der Technik nicht aufweisen.

### Aufgabenstellung

Das der vorliegenden Erfindung zugrunde liegende technische Problem besteht vor allem darin, Verfahren und Mittel zur Herstellung, Gewinnung oder Isolierung von Kollagen oder Matrixproteinen aus kollagenhaltigem Gewebe bereitzustellen, wobei das isolierte Kollagen einen höheren Anteil an Matrixproteinen mit naturidentischer Struktur aufweist. Ein damit verbundenes weiteres technisches Problem besteht darin, verbesserte Biomatrices bereitzustellen, die für die Herstellung von Organ- oder Gewebeersatz oder -äquivalenten oder in vitro Testsystemen geeignet sind. Ein damit verbundenes weiteres technisches Problem besteht darin, in vitro Testsysteme zur Analyse und Diagnose von Infektionen sowie zur Untersuchung und Testung von oder entarteten oder gentechnisch veränderten menschlichen oder tierischen Zellen, Diagnostika und Therapeutika bereitzustellen, die die vorstehend beschriebenen Nachteile im Stand der Technik überwinden.

Das zugrunde liegende technische Problem wird im Wesentlichen gelöst durch ein Verfahren zur Isolierung von Kollagen oder Kollagenmatrixproteinen aus kollagenhaltigem Gewebe nach dem Kennzeichen des Anspruchs 1.

Erfindungsgemäß werden aus dem kollagenhaltigen Gewebe in einem Schritt (a) kollagenhaltige Fasern isoliert und in einem nachfolgenden Schritt (b) die isolierten kollagenhaltigen Fasern in einer wässrigen Harnstofflösung, enthaltend Harnstoff in einer Konzentration von 5 bis 15 mol/l, besonders von 7 bis 12 mol/l, besonders bevorzugt von etwa 9 mol/l, inkubiert, wobei oder wodurch eine kollagenhaltige Fraktion aus den Fasern freigesetzt und herausgelöst wird. Erfindungsgemäß folgt anschließend in einem Schritt (c) das Abtrennen der gelösten kollagenhaltigen Fraktion von Faser- und Geweberesten, sodass eine kollagenhaltige wässrige Lösung erhalten wird. Vorzugsweise wird in einem weiteren Schritt (d) der Harnstoff von der kollagenhaltigen Fraktion abgetrennt und vorzugsweise wird in einem weiteren Schritt (e) die vom Harnstoff befreite kollagenhaltige Fraktion bevorzugt in Pufferlösung renaturiert, sodass renaturiertes isoliertes Kollagen in wässriger Lösung erhalten wird.

Die Erfindung sieht also vor, isolierte kollagenhaltige Fasern im Wesentlichen mit einer hochkonzentrierten Harnstofflösung über einen gewissen Zeitraum zu inkubieren, wobei die die Kollagenmatrixproteine aus dem Gewebe herausgelöst werden. Überraschenderweise erlaubt die erfindungsgemäße Extraktion von die Isolierung überwiegend naturidentischer Matrixproteine, die nach Abtrennen des Harnstoffs in wässrige Pufferlösung quasi vollständig renaturiert werden können. Die zirkuläre Dichroismus-Spektroskopie (DC) der erfindungsgemäß isolierten Kollagenfraktion weist die typische so genannte "Triple-Helices"-Struktur von nativem Kollagen nach, die im DC-Spektrogramm besonders durch ein negatives Band zwischen 217 und 227 nm und gegebenenfalls ein schwächeres negatives Band nahe 200 nm charakterisiert ist. Auch eine UVspektroskopische Untersuchung (UV) der erfindungsgemäß isolierten Kollagenfraktion zeigt eine charakteristische Aminosäure-Signatur von Histidin bei 213 nm, was ebenfalls auf eine intakte native Proteinstruktur hinweist. Vorteilhafterweise eignet sich UV dadurch auch zur einfachen Quantifizierung der Kollagenmatrixproteine, ohne dass dazu die als nachteilig bekannten Antikörpertests, die nachteiligerweise oft Kreuzreaktionen zeigen, eingesetzt werden müssen. Ein Gegenstand der Erfindung ist daher auch ein Verfahren zur Isolierung von Kollagen beziehungsweise Kollagenmatrixproteinen, worin die Konzentrationsbestimmung des Kollagens durch UV erfolgt.

Vorteilhafterweise kann mit dem erfindungsgemäß isolierten Kollagen eine Biomatrix und daraus ein Gewebe- oder Organersatz oder -äquivalent oder ein in vitro Testsystem erhalten werden, das die bekannten Nachteile der, insbesondere eindimensionalen, Schrumpfung nicht mehr aufweist. Die Proliferation von gewebespezifischen Zellen und die Neusynthese von Matrix durch die Zellen aus der Biomatrix erfolgt verbessert oder zumindest im gleichen Maße wie bei mit bekannten Verfahren erhältlichen Biomatrices.

In einer bevorzugten Ausführungsform des Verfahrens ist das eingesetzte kollagenhaltige Gewebe, woraus in Schritt (a) die kollagenhaltigen Fasern isoliert werden, aus Rattenschwänzen isolierte Rattenschwanzsehnen. In einer alternativen Variante ist das kollagenhaltige Gewebe ein, bevorzugt azellularisierter, Schweinedünndarm.

Bevorzugt wird in Schritt (b) die Harnstofflösung mit den kollagenhaltigen Fasern gerührt. Bevorzugt werden in Schritt (b) die kollagenhaltigen Fasern mit der Harnstofflösung für 12 bis 36 Stunden, besonders bevorzugt für etwa 24 Stunden, vorzugsweise unter Rühren, inkubiert, sodass die kollagenhaltige Fraktion besonders effektiv aus den Fasern herausgelöst wird.

Vorzugsweise wird in Schritt (c) die gelöste kollagenhaltige Fraktion von Faser- und Geweberesten durch mechanische Trennverfahren in an sich bekannter Weise, bevorzugt durch Zentrifugieren, abgetrennt. In einer anderen bevorzugten Variante wird alternativ oder zusätzlich durch Filtrieren abgetrennt.

Für bestimmte Anwendungsgebiete ist es zweckmäßig, in Schritt (c) die gelöste kollagenhaltige Fraktion durch Fraktionierung mittels Gelfiltration abzutrennen und gegebenenfalls aufzutrennen. Anschließend können gegebenenfalls mehrere der aufgetrennten Kollagenfraktionen wieder vereinigt werden. Der Fachmann kann so die für das jeweilige Anwendungsgebiet günstige und/oder gewebespezifische Zusammensetzung der Matrixproteine wählen.

Bevorzugt wird die isolierte kollagenhaltige Fraktion beziehungsweise die mittels Fraktionierung aufgetrennten Kollagenfraktionen Analyseverfahren unterzogen. Die Charakterisierung der Matrixproteine findet bevorzugt durch DC statt. In einer weiteren bevorzugten Variante findet die Charakterisierung alternativ oder zusätzlich durch UV statt. In einer weiteren bevorzugten Variante findet die Charakterisierung alternativ oder zusätzlich durch ESI-MS/MS statt. In einer weiteren bevorzugten Variante findet die Charakterisierung alternativ oder zusätzlich durch MALDI-TOF/TOF statt. Die Proteinfraktionen können in an sich bekannter Weise durch eindimensionale beziehungsweise zweidimensionale Gelelektrophoresen, isoelektrische Fokussierung und/oder SDS-PAGE aufgetrennt und isoliert werden.

Um den in der Lösung enthaltenen Harnstoff von der kollagenhaltigen Fraktion zu trennen, wird in Schritt (d) eine Gradientendialyse durchgeführt. Vorzugsweise wird dabei in der Kälte, das heißt bevorzugt bei Temperaturen von 0°C bis etwa 10°C, bevorzugt bei etwa 4°C, und für einen Zeitraum von 4 bis 12 Tagen, vorzugsweise von 7 Tagen, dialysiert. Vorzugsweise wird gegen Wasser dialysiert. Vorzugsweise weist die so hergestellte wässrige Kollagenlösung eine Kollagenkonzentration von etwa 3 bis etwa 8 mg/l auf.

In einer Variante der Erfindung werden die dialysierten Fraktionen auf einen bestimmten Kollagengehalt aufkonzentriert, vorzugsweise um den Faktor 2 oder 3. Die erhaltenen Konzentrate eignen sich besonders für die anschließende Auftrennung im SDS-PAGE, zur weiteren Charakterisierung sowie zur Herstellung kochfester Biomatrices.

Erfindungsgemäß wird die Kollagenfraktion anschließend in dem weiteren Schritt (e) in Phosphat gepufferter Saline (PBS) oder ähnlichen Medien oder Puffersystemen renaturiert. Durch die Renaturierung entwickeln die Matrixproteine im Wesentlichen alle natürlichen Eigenschaften und nativen Strukturparameter zurück und können so zur Herstellung verbesserter Biomatrices eingesetzt werden.

Gegenstand der Erfindung ist auch ein isoliertes Kollagen, welches mittels des erfindungsgemäßen Verfahrens herstellbar ist oder damit hergestellt wird. Die erfindungsgemäß erhältliche wässrige Kollagenlösung enthält einen hohen Anteil an nicht denaturiertem, nativem Kollagen in wässrigem Medium, besonders einen Anteil am Gesamtkollagen in Lösung von ≥ 50%, insbesondere ≥ 60%, ≥ 70%, ≥ 80%, ≥90% oder ≥ 95 %, vorzugsweise ≥ 99%. Ein solches Kollagen weist vorzugsweise in der zirkulären Dichroismus-Spektroskopie die charakteristische Triple Helices-"Signatur" der Triple Helices-Struktur (siehe oben) auf.

In einer weiteren bevorzugten Ausführungsform der Erfindung wird die erhaltenen kollagenhaltige Fraktion wässrige Lösung lyophilisiert, sodass ein lagerfähiges trockenes Kollagenpräparat erhalten wird. Wird das so erhaltene lyophilisierte Kollagen anschließend mit Wasser oder einem geeigneten Medium versetzt, entsteht vorteilhafterweise eine nahezu vollständig native dreidimensionale Struktur des Kollagens. Eine Zerstörung der natürlich vorkommenden Triple-Helix-Struktur durch die Lyophilisierung erfolgt nicht. Ein weiterer Gegenstand der Erfindung ist auch ein erfindungsgemäß erhältliches lyophilisiertes Kollagen. Die wässrige Kollagenfraktion wird dazu in an sich bekannter Weise lyophilisiert, sodass ein trockenes, lagerfähiges Kollagenpräparat erhalten wird. Selbstverständlich ist es auch möglich, die Lösung in gefrorenem Zustand zwischenzulagern, zum Beispiel bei -10°C bis -80°C, vorzugsweise bei etwa -20°C.

Ein weiterer Gegenstand der Erfindung ist ein Verfahren zur Herstellung einer Biomatrix, welches dadurch gekennzeichnet ist, dass zunächst die Schritte (a) bis (e) des erfindungsgemäßen Verfahrens durchgeführt werden, wodurch eine wässrige Kollagenlösung oder kollagenhaltige Fraktion erhalten wird, und in einem weiteren Schritt (f) die erhaltene Kollagenlösung mit Zellkulturmedium oder ähnlichem in einem Verhältnis von 2:1 bis 1:2, vorzugsweise 1:1, zu einer kollagenhaltigen Matrixvorläuferlösung vermischt werden. Der Kollagengehalt der Lösung beträgt vorzugsweise 3 bis 8 mg Kollagen pro ml Lösung, bevorzugter 5 bis 7 mg Kollagen pro ml Lösung. In einem weiteren Schritt (g) wird die so erhaltene Matrixvorläuferlösung, vorzugsweise bei erhöhter Temperatur, bevorzugt bei 21°C bis 37 °C, zu einer kollagenhaltigen Biomatrix geliert. Alternativ wird das Kollagengel durch Auflösen von erfindungsgemäß erhältlichen lypophilisierten Kollagenpräparaten erhalten.

Ein weiterer Gegenstand der Erfindung ist demgemäß auch eine Biomatrix, die nach dem vorgenannten Verfahren herstellbar ist oder bevorzugt damit hergestellt wird. Ein weiterer Gegenstand der Erfindung ist auch eine Biomatrix, welche das mittels des erfindungsgemäßen Verfahrens isolierte Kollagen enthält oder bevorzugt daraus besteht. Die Erfindung betrifft vor allem eine, vorzugsweise gelartige, Biomatrix, zur Verwendung in Kultivierungsverfahren, beispielsweise zur Kultivierung von Zellen eines bestimmten Gewebetyps oder Zellen mehrerer Gewebetypen. Die erfindungsgemäß vorgesehene Kombination aus Biomatrix und darin kultivierten Zellen kann zur Herstellung eines in vitro- Gewebe- oder Organtestsystems verwendet werden.

Unter einer "Biomatrix" wird eine Gelstruktur verstanden, die Kollagen, Zellkulturmedium, Puffer und gegebenenfalls Serum enthält. Bevorzugte Zellkulturmedien sind DMEM (Dulbecco's Modified Eagle Medium) und M199. Ein bevorzugtes Puffersystem ist Hepes-Puffer. Als Serum wird vorzugsweise fötales Kälberserum (FCS) oder humanes insbesondere autologes Serum verwendet. Der pH-Wert der Lösung aus Zellkulturmedium, Puffer und Serum beträgt in bevorzugter Ausführung 7,5 bis 8,5, beispielsweise 7,6 bis 8,2, insbesondere 7,8. Selbstverständlich kann die Biomatrix je nach Anwendungsgebiet weitere Faktoren, beispielsweise Wachstumsfaktoren, Protesglykane, Glucosaminglykane, Adhäsionsmittel, Antibiotika, Selektionsmittel und andere extrazelluläre Matrixkomponenten enthalten.

Zur Herstellung einer Zellen enthaltenden Biomatrix wird ein, vorzugsweise mehrfach (x-fach) konzentriertes, Zellkulturmedium, Serum und Puffer mit vorkultivierten Zellen versetzt, wobei vorzugsweise 1 x bis 2 x 10⁵ Zellen pro ml, bevorzugt 1,5 x 10⁵ Zellen pro ml, verwendet werden. Anschließend wird, beispielsweise im Verhältnis 1:2 bis 2:1, mit der erfindungsgemäß erhältlichen Kollagenlösung in der Kälte bei 0 bis 10°C, insbesondere bei 4°C, gemischt. Das Mischungsverhältnis (Volumen) von Kollagenlösung zu Zellsuspension (Puffer, Serum, Zellen, Kulturmedium) beträgt vorzugsweise 1:1, wobei bei x-fach konzentrierter Gellösung besonders ein Volumenverhältnis von (x-1):1 Kollagenlösung zu Gellösung bevorzugt wird. Anschließend wird die Suspension in Kulturgefäße pipettiert und nach Gelieren bei erhöhter Temperatur, vorzugsweise bei 37°C, das in der Regel innert weniger Minuten passiert, mit Medium überschichtet (Submers-Kultur). Die Biomatrix kann beispielsweise 2 Tage kultiviert werden. Anschließend können noch zusätzlich Zellen anderer Gewebetypen darauf angesiedelt und kultiviert werden.

Eine bevorzugte Ausführungsform der Erfindung umfasst die Kultivierung tierischer oder humaner Zellen in einer dreidimensionalen gelartigen Biomatrix zur Vermehrung dieser Zellen und zur Herstellung eines dreidimensionalen tierischen oder humanen in vitro-Gewebe- oder Organtestsystems. Die mit den erfindungsgemäßen Gewebe- oder Organtestsystemen erzielten Ergebnissen können aussagekräftiger als die mit Tierversuchen ermittelten Ergebnisse sein und eine bessere Übertragbarkeit auf den Menschen gewährleisten. Ein Gegenstand der Erfindung ist auch ein Gewebeäquivalent oder -ersatz, welcher die erfindungsgemäße Biomatrix sowie vitale Gewebezellen, insbesondere gewebespezifische Zellen, welche bevorzugt in und auf oder in oder auf der Biomatrix kultiviert sind, enthält. Ein Gegenstand der Erfindung ist auch ein Organäquivalent oder -ersatz, enthaltend die erfindungsgemäße Biomatrix sowie vitale Gewebezellen, insbesondere organspezifische Zellen, die vorzugsweise in und auf oder in und auf der Biomatrix kultiviert sind. In einer besonders bevorzugten Ausführungsform umfasst die Erfindung die Kultivierung humaner dermaler Fibroblasten in der Biomatrix zur Herstellung eines aus Dermisäquivalent und Epidermisäquivalent bestehenden dreidimensionalen humanen in vitro Hautäquivalentes.

Unter "Kultivieren von Zellen" wird ein, vorzugsweise in vitro stattfindendes, Aufrechterhalten der gewebetypischen Lebensfunktionen von Zellen, beispielsweise von Fibroblasten, in einer geeigneten Umgebung, beispielsweise unter Zu- und Abfuhr von Stoffwechseledukten und -produkten, insbesondere auch eine Vermehrung der Zellen. Im Zusammenhang mit der vorliegenden Erfindung werden unter dermalen Fibroblasten natürlicherweise insbesondere in der Dermis vorkommende oder gentechnisch veränderte Fibroblasten oder deren Vorläufer verstanden. Fibroblasten stellen die Vorläufer dermaler Fibrocyten dar. Die Fibroblasten können tierischer oder menschlicher Herkunft sein; es können aus Gewebe frisch isolierte Zellen oder Zellen primärer oder gentechnisch veränderter oder transformierter Zelllinien wie WI-38, NIH/3T3, MRC-5 sein. Die Biomatrix enthält die zu kultivierenden Fibroblasten und ein aus der erfindungsgemäß erhältlichen Kollagenlösung neu konstituiertes Kollagengerüst einer Konzentration von 2 bis 5 mg, vorzugsweise 3,5 bis 4,5 mg Kollagen pro ml Biomatrix. Das Kollagengerüst wird aus einer erfindungsgemäß erhältlichen, vorzugsweise zellfreien, Lösung von Kollagen gewonnen, wobei die Proteinkonzentration der Kollagenlösung vorzugsweise 5 bis 7 mg/ml beträgt. Zur Herstellung einer beispielsweise Fibroblasten-haltigen Biomatrix wird die Kollagenlösung in der Kälte, vorzugsweise bei 4°C, mit einer Zelllösung oder - suspension, enthaltend ein, vorzugsweise fünffach konzentriertes, Zellkulturmedium, Puffer, vorzugsweise Hepes-Puffer, Serum, vorzugsweise fötales Kälberserum (FCS), und Chondroitin-(4/6)-sulfat, und Fibroblasten, vorzugsweise in einer Konzentration von etwa 1,5 x 10⁵/ml, versetzt und gut gemischt. Dieses Gemisch wird beispielsweise durch Erhöhung der Temperatur auf Raumtemperatur oder 37°C in etwa 2 Minuten geliert. Nach dem Gelieren der Gele wird vorzugsweise Fibronectin auf die Gele gegeben. Fibronectin vermittelt die Bindung der Zellen an Makromoleküle, beispielsweise Kollagen und die Anheftung an Nachbarzellen. Die anschließende Kultivierung der Fibroblasten im Kollagengel erfolgt vorzugsweise in Submers-Kultur; darunter wird verstanden, dass die Zellen mit einer Nährlösung bedeckt sind. Die Fibroblasten enthaltende Biomatrix wird mit Zellkulturmedium überschichtet und bei 37°C und 5% CO₂ unter Standardbedingungen in an sich bekannter Weise kultiviert.

In einer vorteilhaften Ausführungsform der Erfindung können die in der Biomatrix kultivierten Fibroblasten wieder aus der Biomatrix herausgelöst und gegebenenfalls erneut in eine Biomatrix eingebracht werden, wobei die Zellen nach Herauslösen ihre spezifischen Stoffwechselleistungen und ihren Differenzierungsstatus nicht verlieren.

Das erfindungsgemäße Verfahren gestattet es also, eine Zwischenkultivierung der Fibroblasten in der Biomatrix durchzuführen. Bei einer geringen Ausgangsmenge an Fibroblasten bietet das erfindungsgemäße Verfahren daher den Vorteil, dass genügend Zellmaterial für die Herstellung von Dermisäquivalenten und/oder Hautäquivalenten zur Verfügung gestellt werden kann.

Eine weitere vorteilhafte Ausgestaltung der Erfindung sieht vor, dass dermale Fibroblasten, die in ihrer Funktion, ihrer Morphologie und/oder ihrem Differenzierungsstatus. überprüft werden sollen, in eine vorstehend genannte dreidimensionale Biomatrix eingebracht, kultiviert und dabei und/oder danach überprüft werden. Die Erfindung betrifft daher auch unter Verwendung dermaler Fibroblasten durchgeführte Screening- und Diagnoseverfahren, wobei die Fibroblasten gemäß dem vorstehend beschriebenen Verfahren kultiviert und dabei und/oder anschließend untersucht werden können, zum Beispiel auf pharmakologische, onkologische, toxikologische, physiologische, morphologische und/oder molekularbiologische Parameter. Diese Biomatrix enthält neben den zu kultivierenden Fibroblasten ein aus einer Kollagenlösung konstituiertes Gerüst aus menschlichem oder tierischen Kollagen, also gewebetypische Matrixproteine. Dieses Kollagen-Fibroblasten-Gel wird erfindungsgemäß bevorzugt einer einbis zweitägigen Submers-Kultur unterworfen.

In einer weiteren vorteilhaften Ausführungsform der Erfindung werden die dermalen Fibroblasten in der dreidimensionalen Biomatrix, wie vorstehend beschrieben, so kultiviert, dass anschließend ein Dermisäquivalent gewonnen werden kann. Im Zusammenhang mit der vorliegenden Erfindung wird unter einem "Dermisäquivalent" eine Bindegewebe-artige Schicht aus Kollagen und Fibroblasten verstanden, die weitgehend der nativen Dermis entspricht. Dazu werden ein bis drei Tage, vorzugsweise zwei Tage, nach der vorstehend beschriebenen Inkubation der Gele Keratinocyten, Stammzellen der Haut oder Vorläuferzeellen der Keratinocyten, vorzugsweise vorkultivierte, undifferenzierte Keratinocyten-Stammzellen, aus bevorzugt humanem Biopsiegewebe auf das Gel ausgesät, mit KBM-Medium überschichtet und einer ein- bis dreitägigen Submers-Kultivierung unterworfen. Eine vollständige Differenzierung der Keratinocytenschichten wird durch eine Airlift-Kultur in etwa 1,8 mmol/l CaCl₂ enthaltendem KBM-Medium (ohne hEGF und BPE) erreicht. Im Zusammenhang mit der vorliegenden Erfindung wird unter einer "Airlift-Kultur" eine Kultur verstanden, wobei die Höhe des Nährmedienspiegels genau auf die Höhe der Biomatrix abgestimmt ist, während die Keratinocyten oder durch die Keratinocyten gebildeten Zellschichten über dem Nährmedienspiegel liegen und vom Nährmedium nicht bedeckt werden, das heisst die Kultivierung erfolgt an der Grenzschicht Luft-Nährmedium, wobei die Versorgung der Kulturen von unten her erfolgt. Dazu werden die Inserts aus der Mikrotiterplatte mit 24 Vertiefungen in die Vertiefungen einer Mikrotiterplatte mit 6 Vertiefungen mit jeweils einem Durchmesser von 3,5 cm übertragen. Nach einer, vorzugsweise 12- bis 14-tägigen Airlift-Kultur entwickelt sich ein hauttypisches, aus Dermisäquivalent und Epidermisäquivalent bestehendes in vitro-Vollhautmodell, welches für die erfindungsgemäßen Testverfahren vorteilhaft eingesetzt werden kann.

Auf die Fibroblasten enthaltende Biomatrix werden danach Keratinocyten oder Keratinocyten-Stammzellen ausgesät. Unter "Keratinocyten" werden Zellen der Epidermis, die verhornendes Plattenepithel bilden, oder gentechnisch veränderte Keratinocyten oder deren Vorläufer verstanden, die tierischer oder menschlicher Herkunft sein können. Da die Ausbildung einer gut differenzierten Epidermis mit intakter Verhornung in starkem Maße vom Anteil basaler Stammzellen in den verwendeten Keratinocyten abhängt, handelt es sich bei den auf das Kollagengel ausgesäten Keratinocyten vorzugsweise um möglichst undifferenzierte Keratinocyten-Stammzellen aus humanem Biopsiegewebe, das heisst Cytokeratin 19- beziehungsweise Integrin β1-positive basale Stammzellen. Vorzugsweise handelt es sich dabei um vorkultivierte Zellen, besonders bevorzugt um Keratinocyten in der ersten oder in der zweiten Zellpassage.

In besonders bevorzugter Ausführungsform werden vorzugsweise Keratinocyten mit einem vergleichsweise hohen Anteil, beispielsweise 0,5%, 1%, 2%, 5%, 8%, oder 10% an der Keratinocyten-Zellpopulation, oder umfassend nur undifferenzierte Stammzellen, eingesetzt. Unter Verwendung spezifischer Kulturbedingungen,'die insbesondere eine mehrtägige Submerskultur und eine nachfolgende mehrtägige Airlift-Kultur des Biomatrix-Systems umfassen, und spezifischer Kulturmedien durchlaufen die Keratinocyten eine Differenzierung zu einer mehrschichtigen Epidermisschicht. Erfindungsgemäß ist darüber hinaus in einer bevorzugten Ausführung vorgesehen, dass vor, während oder nach der Aussaat der Keratinocyten auch andere Zelltypen und/oder andere Zellen anderer Gewebetypen auf der Biomatrix ausgesät werden können, zum Beispiel Immunsystemzellen. Vorteilhafterweise wird erreicht, dass das Dermisäquivalent im Verlauf der Kultivierungsdauer keinem undefinierten Schrumpfungsprozess unterworfen ist.

Die Erfindung betrifft daher auch ein hauttypisches, dreidimensionales, vorzugsweise humanes, in vitro-Hautäquivalent, bestehend aus einem Dermisäquivalent und einem Epidermisäquivalent, und Verfahren zur Herstellung, Kultivierung und Anwendung dieses Hautäquivalents sowie dessen Bestandteile. Hauttypische Vollhautmodelle, die auch als in vitro-Hautäquivalente bezeichnet werden, können insbesondere in der Dermatologie und in der Allergologie als Testhaut verwendet werden, um Substanzen, beispielsweise potentielle Arzneimittel oder Kosmetika, oder Agenzien, wie Licht und Wärme, auf ihre pharmakologischen Wirkungen, insbesondere Reiz-, Toxizitäts- und Entzündungswirkungen, und auf ihre Verträglichkeit zu untersuchen.

Eine weitere Ausführung der Erfindung umfasst die Kultivierung von Darmfibroblasten mit der erfindungsgemäß erhältlichen Biomatrix zur Herstellung eines aus bevorzugt Caco2-Zellen oder auch Darmepithelzellen oder anderer humaner Zelllinien bestehenden dreidimensionalen humanen in vitro-Darmtestsystems. Darmfibroblasten sind natürlicherweise, insbesondere im Darmgewebe, vorkommende oder gentechnisch veränderte Fibroblasten tierischer oder menschlicher Herkunft oder deren Vorläuferzellen. Zur Herstellung der erfindungsgemäßen Darmfibroblasten-haltigen Biomatrix wird die Kollagenlösung im Volumenverhältnis 1:1, vorzugsweise bei 4°C, mit einer auch als Gellösung bezeichneten Lösung, enthaltend ein, vorzugsweise 2-fach konzentriertes, Zellkulturmedium, Puffer, vorzugsweise Hepes-Puffer, und Serum, vorzugsweise 10 %iges Serum, und vorzugsweise 1,5 x 10⁵/ml Darmfibroblasten, insbesondere vorkultivierten Darmfibroblasten, versetzt und gut gemischt. Dieses Gemisch wird durch Erhöhung der Temperatur auf Raumtemperatur oder 37° geliert. Die anschließende Kultivierung der Darmfibroblasten im Kollagengel erfolgt vorzugsweise in Submers-Kultur. Die Fibroblasten enthaltende Biomatrix wird bei 37°C inkubiert.

Ein weiterer Gegenstand der Erfindung ist ein in vitro Testsystem, enthaltend die erfindungsgemäße Biomatrix und vitale Zellen, die in und auf oder in oder auf der Biomatrix kultiviert sind. Die Erfindung betrifft vor allem solche in vitro Testsysteme, die zur Analyse und Diagnose von durch pathogene und/oder parasitäre Mikroorganismen hervorgerufenen Infektionen und/oder Erkrankungen des menschlichen oder tierischen Körpers eingesetzt werden können, in vitro Testsysteme zur Analyse und Diagnose von entarteten menschlichen und tierischen Zellen, in vitro Testsysteme zur Analyse und Diagnose von gentechnisch veränderten menschlichen und tierischen Zellen und in vitro Testsysteme zur Untersuchung und Testung von Antiinfektiva und Arzneimitteln gegen Tumore, insbesondere Cytostatika, sowie dreidimensionale tierische in vitro-Organ- und Gewebemodelle, insbesondere von für Infektionen anfälligen Geweben, wie Darm, Leber, Haut, Cornea, Trachea und Schleimhäuten.

Zu untersuchende Zellen werden bevorzugt in der erfindungsgemäß bereitgestellten dreidimensionalen, gelartigen, bindegewebsähnlichen Biomatrix kultiviert und können sich dort vermehren. Diese Biomatrix enthält die zu kultivierenden Zellen in einem oder auf einem aus einer Kollagenlösung konstituierten Gerüst aus Kollagen, das heißt gewebetypischen Matrixproteinen. Je nach gewünschtem Gewebetyp können zusätzlich weitere andere Zelltypen, vorzugsweise andere Primärzellen, auf der Biomatrix ausgebracht werden. Unter Verwendung spezifischer Kulturbedingungen und spezifischer Kulturmedien können die bevorzugt in der Biomatrix enthaltenen Zellen und die bevorzugt auf die Biomatrix aufgebrachten anderen Zelltypen eine Differenzierung zu einem mehrschichtigen dreidimensionalen tierischen Gewebe- oder Organtestmodell durchlaufen. Eine solche Cokultivierung des erfindungsgemäßen tierischen in vitro-Gewebe- oder Organtestsystems mit einem parasitären oder pathogenen Mikroorganismus bietet die Möglichkeit, sowohl den Infektionsprozess selbst als auch die Abwehrreaktion der entsprechenden organoiden Zellsystemen zu studieren. Beispielsweise können größere Mengen eines infizierten Zellmaterials und des Pathogens selbst gewonnen werden. Das gewonnene Material kann dann mit herkömmlichen histologischen, biochemischen, molekularbiologischen oder immunologischen Verfahren weiter analysiert werden, um beispielsweise morphologische Änderungen infizierter Zellen, die Ausschüttung spezifischer Stoffe durch das Pathogen, wie Toxine oder für auftretende Resistenzen relevante Proteine, oder die Ausschüttung spezifischer Stoffe durch befallene Zellen, wie Interleukine, als Abwehrreaktion genauer zu studieren oder um Transkriptions- und/oder Expressionsprofile zu erstellen, auf deren Basis sich beispielsweise Virulenzfaktoren als Targets für die Entwicklung von Antiinfektiva identifizieren lassen.

Eine bevorzugte Ausführung der Erfindung umfasst auch die Cokultivierung eines erfindungsgemäß hergestellten dreidimensionalen in vitro-Gewebe- oder Organtestsystems mit einem pathogenen oder parasitären Mikroorganismus. Im Zusammenhang mit der vorliegenden Erfindung werden unter "pathogenen oder parasitären Mikroorganismen", hier auch als infektiöse Agenzien bezeichnet, sowohl eukaryontische als auch prokaryontische Mikroorganismen, wie Bakterien, Pilze, Protozoen, Viroide, aber auch Prionen oder Viren, verstanden, die einen Makroorganismus, insbesondere einen menschlichen oder tierischen Organismus, befallen und in oder auf Geweben dieses Organismus leben und zu einer Infektion dieses Organismus führen können, jedoch nicht notwendigerweise dazu führen müssen. Im Zusammenhang mit der Erfindung bedeutet der Begriff "Cokultivierung" ein, vorzugsweise in vitro stattfindendes, gleichzeitiges Aufrechterhalten der Lebensfunktionen von tierischen Zellen und Mikroorganismen in der gleichen, für beide geeigneten Umgebung, beispielsweise unter Zu- und Abfuhr von Stoffwechseledukten und - produkten, insbesondere auch eine gleichzeitige Vermehrung der Zellen und der Mikroorganismen.

In einer weiteren Ausgestaltung der Erfindung wird unter Verwendung eines erfindungsgemäß hergestellten in vitro-Gewebe- oder Organtestsystems, vor allem in Verbindung mit einem Cokultivierungsverfahren, die Wirkung chemischer Substanzen, insbesondere von Antiinfektiva, oder von Agenzien auf den Infektionsprozess beziehungsweise das Wachstum eines pathogenen Mikroorganismus zu untersuchen. Im Zusammenhang mit der Erfindung soll der Begriff "Agens" insbesondere chemische, biologische oder physikalische Mittel, wie Licht oder Wärme, umfassen, die eine potentielle Wirkung auf lebende Zellen ausüben können.

Eine bevorzugte Ausführung der Erfindung umfasst die Analyse entarteter Zellen. Im Zusammenhang mit der Erfindung umfasst der Begriff "entartet" alle Veränderungen einer normalen Zelle, beispielsweise Zellpolymorphie, Anisozytose, Kernpolymorphie, Polychromasie, gestörte Kern-Plasma-relation und Aneuploidie, die zu einer gestörten Differenzierung oder zu einer Entdifferenzierung und zu einem deregulierten Wachstum der Zelle führen können, und betrifft insbesondere Zellen maligner Tumore. Aus entarteten Zellen, insbesondere der vorstehend genannten Gewebe oder Organe, wird ein in vitro-Gwewbe- oder Organtestsystem aufgebaut, um größere Mengen des entarteten Zellmaterials zu gewinnen. Das gewonnene Material wird mit herkömmlichen Verfahren, beispielsweise histologischen, biochemischen, molekularbiologischen oder immunologischen Verfahren, weiter analysiert, um die Ausschüttung spezifischer Stoffe zu untersuchen und Transkriptions- und Expressionsprofile zu erstellen. An dem aus entarteten Zellen aufgebauten in vitro-Gewebe- oder Organtestsystem wird die Wirkung von Arzneimitteln und von potentiell als Arzneimittel geeigneten Substanzen, insbesondere im Hinblick auf deren Fähigkeit zur Hemmung der Zellteilung, untersucht.

In einer bevorzugten Ausgestaltung der Erfindung werden patientenspezifische entartete Zellen zur Etablierung eines in vitro-Gewebe- oder Organtestsystems verwendet, um Therapiemöglichkeiten für die spezielle Tumorerkrankung des Patienten zu untersuchen.

In einer bevorzugten Ausgestaltung der Erfindung ist die Überprüfung gentechnisch veränderter Zellen, insbesondere der vorstehend genannten Gewebe und Organe; vorgesehen. Im Zusammenhang mit der vorliegenden Erfindung umfasst der Begriff "gentechnisch veränderte Zellen" alle Zellen, die mit Hilfe gentechnischer Verfahren manipuliert wurden, wobei entweder Fremd-DNA und/oder -RNA in die Zelle eingeschleust wurde oder die eigene DNA und/oder -RNA, beispielsweise durch Deletionen, Inversionen und Anlagerungen, modifiziert wurde. In einer besonders bevorzugten Ausführungsform ist vorgesehen, im Hinblick auf eine Gentherapie patientenspezifischer Krankheiten gentechnisch veränderte Zellen in vitro zu testen, insbesondere auf deren Funktionalität, wobei ein in vitro-Gewebe- oder Organtestsystem unter Verwendung solcher gentechnisch veränderter Zellen etabliert wird.

Schließlich ist ein weiterer Gegenstand der Erfindung die Verwendung des erfindungsgemäßen Kollagens zur Herstellung eines Gewebeäquivalentes oder -ersatzes, zur Herstellung eines Organäquivalentes oder -ersatzes oder zur Herstellung eines - in vitro-Testsystems.

### Ausführungsbeispiele

Die Erfindung wird durch die nachfolgenden Figuren und Beispiele näher erläutert, ohne dass diese beschränkend zu verstehen sind.

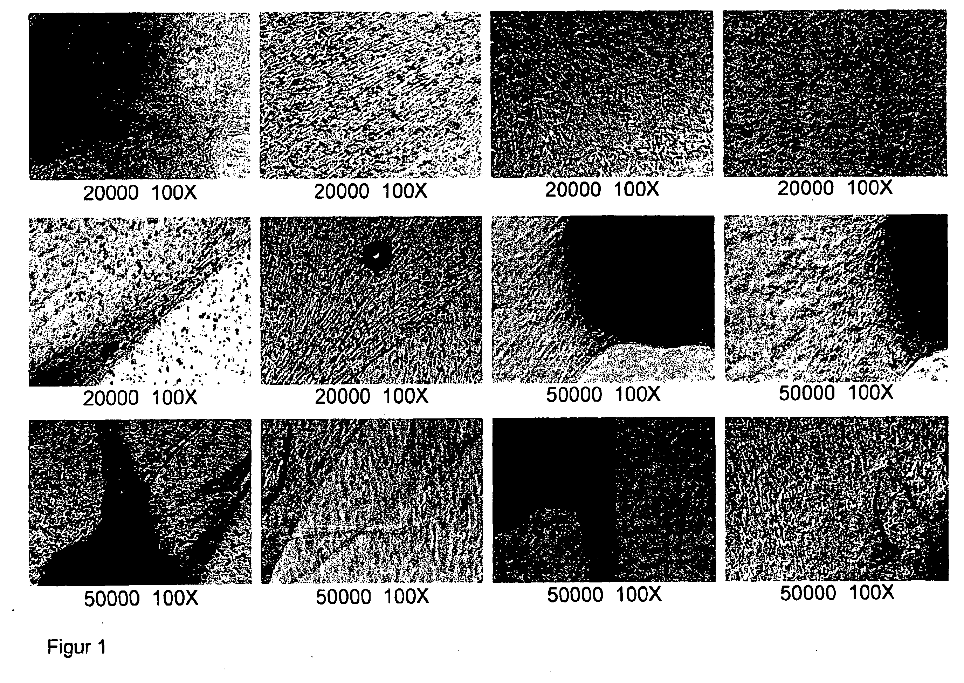

- Figur 1: zeigt die histologische Darstellung von Fibroblastenkul- turen nach 45 Tagen in/auf einer erfindungsgemäß be- reitgestellten Biomatrix.

### Beispiel 1: Isolierung von Kollagen aus kollagenhaltigem Gewebe

### Isolierung der kollagenhaltigen Fasern

Zur Herstellung einer Kollagenlösung wird als kollagenhaltiges Gewebe Sehnen aus Rattenschwänzen verwendet. Alle Arbeiten werden unter sterilen Bedingungen mit sterilen Materialien durchgeführt. Die Rattenschwänze werden nach Lagerung bei -20°C mit 70%-igem Alkohol oberflächlich desinfiziert. Die Haut der Rattenschwänze wird abgezogen und die einzelnen Kollagenfasern werden herausgezogen. Bei Verwendung anderer Ausgangsgewebe können gegebenenfalls vorhandene Zellen schonend durch mechanische, enzymatische oder chemische Behandlung entfernt werden.

Die Kollagenfasern werden in phosphatgepufferter Saline (PBS) (pH 7,2) gesammelt, in 70%-igem Alkohol etwa 10 min oberflächlich desinfiziert und anschließend gründlich mit PBS gewaschen. Das Gewicht der Fasern wird bestimmt.

### Extraktion der Matrixproteine

Zur Extraktion der Matrixproteine werden die Fasern in eine hochkonzentrierte Harnstofflösung überführt; die Endkonzentration beträgt 9 mol/l. Dieser Ansatz wird etwa 24 Stunden bei etwa 4°C gerührt. Anschließend werden die nicht gelösten Kollagenteile mittels Zentrifugation (1000 Upm, 1 Stunde, 8°C) entfernt. Das Kollagen liegt nun in Lösung und nicht in Faser-, Gerüst- oder Matrixform vor.

### Gel-Filtration

Alternativ zur Abtrennung mittels Zentrifugation wird die Kollagenlösung in einem anderen Ansatz einer Gel-Filtration unterzogen. Dazu werden nach der Extraktion etwa 50 ml der harnstoffhaltigen Kollagenlösung auf eine 1,6 I-Säule (Durchmesser: 6 cm, Höhe: 60 cm) mit einer Superose^{™} 12 (GE Healthcare) entsprechenden Packung aufgebracht und mit einer Durchlaufrate von etwa 25 ml/h eluiert. 100 Fraktionen zu je 10 ml werden gesammelt. Je nach gewünschter Proteinzusammensetzung werden mehrere Fraktionen wieder vereinigt, andere werden verworfen.

### Gradientendialyse

Zur Entfernung des Harnstoffs in der Kollagenlösung wird eine Gradientendialyse gegen Wasser durchgeführt. Beginnend mit 9 mol/l Harnstoff wird innerhalb von 7 Tagen bei 4°C auf 0 mol/l Harnstoff in PBS dialysiert. Dabei wird das Kollagen weitestgehend renaturiert wie entsprechende Analysen zeigen.

### Lyophilisierung

In einem weiteren Ansatz wird die der Dialyse entnommene harnstofffreie Kollagenfraktion zur Haltbarmachung in an sich bekannter Weise lyophilisiert.

### Beispiel 2: Charakterisierung der isolierten Matrixproteine

Die Struktur der erfindungsgemäß erhältlichen und mittels Gelfiltration fraktionierten und mittels Gradientendialyse gegen Wasser vom Harnstoff befreiten Kollagenproteine wurden biophysikalisch charakterisiert.

### CD-Spektroskopie

Die zirkuläre Dichroismus-Spektroskopie (CD) dient der Überprüfung der Proteinstrukturen. Es ergeben sich charakteristische Spektren von so genannten "Triple-Helices". Diese zeigen eine typische "Signatur", welche durch ein kleines negatives Band zwischen 217 und 227 nm und einem gegebenenfalls sichtbaren weniger intensiven negativen Band nahe 200 nm charakterisiert ist. Diese Spektren unterscheiden sich deutlich von CD-Spektren von Kollagenproteinen ungeordneter Struktur ("random coil"), α-Helix oder β-Faltblattstrukturen. Die Triple-Helix Struktur ist typisch für Kollagen I, welches in wässriger Lösung dominiert; der größte Teil des isolierten Kollagens faltet sich in Wasser zur ursprünglichen nativen Struktur.

In einem weiteren Ansatz wurde erfindungsgemäß erhältliche Kollagen lyophilisiert und anschließend wieder in Wasser gelöst und der CD-Spektroskopie zugeführt. Es zeigt sich, dass auch aus lyophilisiertem Kollagen hergestellte Kollagenlösung die charakteristische Triple-Helix Struktur aufweist. Lyophilisiertes erfindungsgemäß hergestelltes Kollagen zeigt eine hohe Wasserlöslichkeit, ohne dass die native Struktur des Kollagens dabei irreparabel zerstört wird. Diese Eigenschaft konnte bisher mit Kollagen aus natürlichen Quellen nicht erreicht werden.

### UV-Spektroskopie

Zur Bestätigung der CD-Ergebnisse wurden UV-Spektren der isolierten Kollagenfration im Bereich von 190 bis 320 nm gemessen. Die UV-Spektren bestätigen die Analyse der CD-Spektren. Zwischen verschiedenen Proteinstrukturen besteht ein Gleichgewicht. So zeigt sich, dass sich bei der Verdopplung der Proteinkonzentration der charakteristische Peak des Spektrums halbiert wurde; das Gleichgewicht verschiebt sich zur alternativen Konformation.

In einem Vergleichsexperiment wurde mittels herkömmlicher Essigsäure-Extraktion erhältliche wässrige Kollagenlösung untersucht. Im Gegensatz zum herkömmlichen Essigsäureextrakt zeigt das erfindungsgemäß erhältliche Kollagen charakteristische Aminosäurereste im UV-Spektrum. Dazu zählen vor allem Histidin bei einer Wellenlänge von 213 nm. Dies erlaubt vorteilhafterweise die direkte Konzentrationsbestimmung des erfindungsgemäß erhältlichen Kollagens mittels objektiver spektroskopischer und photometrischer Verfahren.

### Biochemische Charaktierisierung

In einem weiteren Ansatz wurde überprüft, ob das erfindungsgemäß erhältliche Kollage glycosyliert ist. Durch enzymatischen Verdau mittels O-Glucanase wurde die eventuell vorhandene O-Glycosylierung abgespalten. Anschließend wurde das so erhältliche Protein durch eine zweidimensionale Gelelektrophorese aufgetrennt und mit Coomassie gefärbt. Die isoelektrische Fokussierung zeigt, dass das erfindungsgemäß erhältliche Kollagen vor Deglucosylierung im pl-Bereich (isoelektrischer Punkt) zwischen 4,5 und 6 lokalisiert. Der theoretisch zu erwartende Wert liegt bei 5,5. Nach Deglucosylierung zeigt sich ein charakteristischer "Schmier", der auf die abgespalteten Zucker hinweist. Trotz Deglycosylierung bleibt der isoelektrische Punkt der Proteine erhalten, das Molekulargewicht reduziert sich entsprechend.

### Beispiel 3: Herstellung einer Biomatrix

16 ml Kollagenlösung werden in ein 50 ml-Zentrifugenröhrchen gegeben und auf Eis gestellt. Jeweils 600 µl werden vorsichtig in die Vertiefung einer Mikrotiterplatte mit 24 Vertiefungen (Durchmesser je Vertiefung 10 mm) gegossen. Durch eine 2-minütige Inkubation bei 37°C erfolgt eine Gelierung des Gemisches.

Vor der Aussaat von darauf zu kultiverenden Zellen wird zunächst vorsichtig das Medium in den Vertiefungen der Mikrotiterplatte und von den Gelen abgesaugt.

### Beispiel 4: Herstellung einer Fibroblastenkultur

Zur funktionellen Charakterisierung der gemäß Beispiel 3 hergestellten Biomatrix wurden Fibroblasten auf der Matrix kultiviert. Als Vergleichsansatz wurden nach bekannten Verfahren (EssigsäureExtraktion) hergestellte Matrices eingesetzt.

Zur Gewinnung der Fibroblasten wurde ein an sich bekanntes Verfahren eingesetzt. Im Einzelnen wurde humane Spenderhaut (Präpuzien) mit Dispase-Lösung inkubiert und anschließend mit Trypsin-Lösung behandelt. Von den enzymatisch vorbehandelten Gewebestückchen wurde die Epidermisschicht abgezogen, die Dermis anschließend mit einem Skalpell zerkleinert und für 30 bis 45 Minuten in Kollagenase Typ 4 (500 U/ml) bei 37°C inkubiert. Anschließend wurde bei 1000 rpm für 5 Minuten unter Absaugen des Überstandes zentrifugiert. Die Pellets wurden in DMEM + 10% FCS resuspendiert und erneut zentrifugiert. Die Pellets wurden nach Absaugen des Überstandes in 2 ml DMEM + 10% FCS aufgenommen und in eine unbeschichtete Zellkulturflasche überführt. Nach einer Kultivierung von ein bis zwei Tagen wurden zusätzlich 10 bis 15 ml DMEM + 10% FCS zugefügt und weiter kultiviert. Alle drei Tage wurde ein Medienwechsel durchgeführt. Die Kultivierung erfolgte in einem Wasserdampf-gesättigten Brutschrank unter üblichen Zellkulturbedingungen (37°C und 5% CO₂-Atmosphäre)

Im Vergleichsansatz werden die Fibroblasten passagiert und in einer Konzentration von 1 x 10⁴ Zellen/ml in einer Gelgießlösung (bestehend aus Zellkulturmedium, Serum und Puffer) aufgenommen und in eine mittels Essigsäureextraktion erhältliche Kollagenlösung einpipettiert. Mit Hilfe einer Pipette/Spritze wurde das Gemisch aufgenommen, gleichzeitig gemischt und je 500 µl in eine 24-Well-Platte pipettiert. Danach erfolgte die Inkubation der Gele bei 37°C für 5 Minuten in einem Brutschrank, um das Ausgelieren der Gele zu ermöglichen. Nach Ausgelieren der Gele wurden diese mit cirka 1,5 ml/Well DMEM + 10% FCS + 1% Gentamycin versetzt und bei Standardzellkulturbedingungen kultiviert. Nach einer Woche wurde im Vergleichsansatz eine starke Schrumpfung der Kulturen beobachtet. Nach einer Kultivierungsdauer von etwa 18 Tagen mussten die Experimente im Vergleichsansatz wegen zu starker Schrumpfung abgebrochen werden.

Im Gegensatz dazu wurde im erfindungsgemäßen Ansatz die erfindungsgemäß erhältliche Biomatrix zunächst ausgeliert und anschließend wurde darauf die Zellen nach Passagieren in einer Konzentration von 1 x 10⁴ Zellen/ml in Gelgießlösung als Fibroblasten-Suspension gegeben. Dann wurde in an sich bekannter Weise (siehe Vergleichsexperiment) kultiviert. Nach über 40 Tagen Kultivierungsdauer konnte durch Vitalfärbung bestätigt werden, dass die Fibroblasten gut gewachsen waren. Durch mikroskopische Beobachtung und histologische Verfärbungen (Hämatoxolin-losin) konnte dieses Wachstum auch nach 60 Tagen eindeutig beobachtet und sichtbar gemacht werden. Aus der histologischen Färbung ergibt sich auch, dass die Fibroblasten aus der Biomatrix neue Matrix synthetisiert haben (Figur 1).

### Beispiel 5: Herstellung eines mehrschichtigen in vitro Hautmodells

Es wurde ein Hautmodell, bestehend aus humanen Fibroblasten und primären Keratinozyten hergestellt.

Die humanen Fibroblasten wurden wie in Beispiel 4 gewonnen. Die Gewinnung der Keratinozyten erfolgt wie folgt: Die aus dem enzymatisch behandelten Gewebestückchen abgetrennte Epidermis wurde einer weiteren Trypsin-Behandlung unterzogen und anschließend mechanisch zerkleinert. Die mechanisch zerkleinterten Epidermispartikel wurden von einem speziellen Keratinozytenmedium aufgenommen und/oder mit Trypsininhibitor abgestoppt. Die Zellen werden anschließend bei 1000 rpm für 5 Minuten zentrifugiert, der Überstand wird verworfen und die Pellets vorsichtig mit 2 ml Keratinozytenmedium resuspendiert und in ein Kulturgefäß überführt. Nach etwa 4 Stunden wird unter sterilen Bedingungen ein Medienwechsel vorgenommen. Der Medienwechsel erfolgt anschließend alle zwei Tage. Die Kultivierung erfolgt in an sich bekannter Weise unter Standardkultivierungsbedingungen (siehe Beispiel 4).

Zur Herstellung des in vitro-Hautmodells wurden die erfindungsgemäß erhältliche Kollagenlösung (siehe Beispiel 1) und eine so genannte Gelgießlösung, bestehend aus Zellkulturmedium, Serum und Puffer, vorgelegt und auf Eis gestellt. Zunächst werden die Fibroblasten passagiert und in einer Konzentration von etwa 1 x 10⁴ Zellen/ml in der Gelgießlösung aufgenommen und sofort luftblasenfrei in die Kollagenlösung einpipettiert. Mit Hilfe einer Pipette/Spritze wird das Gemisch aufgenommen und dabei gleichzeitig gemischt. Je 500 µl werden in eine 24-Well-Platte mit speziellem Insert (Fa. Nunc) pipettiert. Danach erfolgt die Inkubation der Gele bei 37°C für 5 Minuten in einem Brutschrank. Nach Ausgelieren der Gele werden diese mit cirka 1,5 ml DMEM + 10% FCS + 1 % Gentamycin pro Well versetzt und über Nacht bei 37°C inkubiert. Am nächsten Tag erfolgt das Absaugen des Mediums. Auf das Gel werden nun 25 ml Fibronektin in einer Konzentration von 50 µg/mg pipettiert und das Gel erneut für etwa 30 Minuten in den Brutschrank gestellt.

Danach erfolgt die Aussaat der Keratinozyten. Die Keratinozyten werden dazu aus der vorgenannten Kultur passagiert und je 1 x 10⁵ Zellen/ml in 50 µl/KMBr + 5% FCS auf die Gele pipettiert. Anschließend wird für 20 Minuten bei 37°C inkubiert. Danach werden die Gele mit KMBr + 5% FCS in einer Submers-Kultur weiterkultiviert. In den nachfolgenden fünf Kultivierungstagen wird die FCS-Konzentration von 5% auf 0% reduziert. Danach erfolgt die Weiterkultivierung der Präparate für 12 bis 14 Tage in luftexponierter Umgebung.

## Patentansprüche

1. Verfahren zur Isolierung von Kollagen oder Kollagenmatrixproteinen aus kollagenhaltigem Gewebe, umfassend die Schritte:
a) Isolieren von kollagenhaltigen Fasern aus dem Gewebe;
b) Inkubieren der isolierten kollagenhaltigen Fasern in einer wässrigen Lösung, enthaltend Harnstoff in einer Endkonzentration von 5 bis 15 mol/l, wobei eine kollagenhaltige Fraktion aus den Fasern herausgelöst wird;
c) Abtrennen der gelösten kollagenhaltigen Fraktion von Faser- und Geweberest;
d) Abtrennen des Harnstoffs von der kollagenhaltigen Fraktion mittels Gradientendialyse, so dass eine kollagenhaltige wässrige Lösung erhalten wird; und
e) Renaturieren des Kollagens in Phosphat gepufferter Saline (PBS), wobei eine Kollagenlösung mit isoliertem Kollagen erhalten wird.

2. Verfahren nach einem der vorhergehenden Ansprüche, wobei das kollagenhaltige Gewebe isolierte Rattenschwanzsehnen ist.

3. Verfahren nach einem der vorhergehenden Ansprüche, wobei das kollagenhaltige Gewebe azellularisierter Schweinedünndarm ist.

4. Verfahren nach einem der vorhergehenden Ansprüche, wobei in Schritt b) die Harnstoffendkonzentration 7 bis 12 mol/l beträgt.

5. Verfahren nach einem der vorhergehenden Ansprüche, wobei in Schritt b) die Harnstoffendkonzentration 9 mol/l beträgt

6. Verfahren nach einem der vorhergehenden Ansprüche, wobei in Schritt b) die kollagenhaltigen Fasern mit der Harnstofflösung gerührt werden.

7. Verfahren nach einem der vorhergehenden Ansprüche, wobei in Schritt b) die kollagenhaltigen Fasern mit der Harnstoff lösung für 12 bis 36 Stunden inkubiert werden.

8. Verfahren nach einem der vorhergehenden Ansprüche, wobei in Schritt c) das Abtrennen der gelösten kollagenhaltigen Fraktion durch Zentrifugieren und/oder Filtrieren erfolgt.

9. Verfahren nach einem der vorhergehenden Ansprüche, wobei in Schritt c) das Abtrennen der gelösten kollagenhaltigen Fraktion durch Fraktionierung mittels Gelfiltration erfolgt und anschließend gegebenenfalls mehrere Fraktionen wieder vereinigt werden.

10. Verfahren nach einem der vorhergehenden Ansprüche, wobei in Schritt d) in der Kälte für 4 bis 12 Tage gegen Wasser dialysiert wird.

11. Verfahren nach einem der vorhergehenden Ansprüche, wobei die erhaltene wässrige Kollagenlösung einen Kollagengehalt von 3 bis 8 mg/ml aufweist

12. Verfahren nach einem der vorhergehenden Ansprüche, wobei in einem weiteren Schritt die erhaltene Kollagenlösung lyophilisiert wird.

13. Verfahren nach einem der vorhergehenden Ansprüche, wobei der Kollagengehalt der erhaltenen wässrigen Kollagenlösung durch UV-Spektroskopie (UV) bestimmt wird.

14. Verfahren nach Anspruch 13, wobei zur Konzentrationsbestimmung im UV-Spektrogramm die charakteristische Aminosäure-Signatur von Histidin bei 213 nm ausgewertet wird.

15. Isoliertes Kollagen, herstellbar oder hergestellt nach dem Verfahren nach einem der vorstehenden Ansprüche.

16. Kollagen nach Anspruch 15, das in der zirkulären Dichroismus-Spektroskopie (DC) eine Triple-Helices-Signatur zeigt.

17. Kollagen nach Anspruch 16, wobei die Triple-Helices-Signatur im DC-Spektrogramm charakterisiert ist durch ein negatives Band zwischen 217 und 227 nm und gegebenenfalls ein schwächeres negatives Band nahe 200 nm.

18. Kollagen nach einem der Ansprüche 15 bis 17, das in der UV-Spektroskopie (UV) eine charakteristische Aminosäure-Signatur von Histidin bei 213 nm zeigt.

19. Verfahren zur Herstellung einer Biomatrix, umfassend die Schritte:
Durchführen der Schritte a) bis e) des Verfahrens nach einem der Ansprüche 1 bis 14;
f) Vermischen der erhaltenen Kollagenlösung mit Zellkulturmedium in einem Verhältnis von 2:1 bis 1:2 zu einer kollagenhaltigen Matrixvorläulösung; und
g) Gelieren der Matrixvorläuferlösung zu einer kollagenhaltigen Biomatrix bei erhöhter Temperatur.

20. Biomatrix, herstellbar oder hergestellt nach dem Verfahren nach Anspruch 19.

21. Biomatrix enthaltend isoliertes Kollagen nach einem der Ansprüche 15 bis 18.

22. Gewebeersatz, enthaltend die Biomatrix nach Anspruch 20 oder 21 und vitale Gewebezellen, die in und/oder auf der Biomatrix kultiviert sind.

23. Organersatz, enthaltend die Biomatrix nach Anspruch 20 oder 21 und vitale Gewebezellen, die in und/oder auf der Biomatrix kultiviert sind.

24. In vitro Testsystem, enthaltend die Biomatrix nach Anspruche 20 oder 21 und vitale Zellen, die in und/oder auf der Biomatrix kultiviert sind.

25. Verwendung des Kollagens nach einem der Ansprüche 15 bis 18 oder der Biomatrix nach Anspruch 20 oder 21 zur Herstellung eines Gewebeersatzes.

26. Verwendung des Kollagens nach einem der Ansprüche 15 bis 18 oder der Biomatrix nach Anspruch 20 oder 21 zur Herstellung eines Organersatzes.

27. Verwendung des Kollagens nach einem der Ansprüche 15 bis 18 oder der Biomatrix nach Anspruch 20 oder 21 zur Herstellung eines in vitro Testsystems.

## Claims

1. Method for the isolation of collagen or collagen matrix proteins from collagen-containing tissue, comprising the steps:
a) isolating collagen-containing fibres from the tissue;
b) incubating the isolated collagen-containing fibres in an aqueous solution, comprising urea in an end concentration of 5 to 15 mol/l, a collagen-containing fraction being dissolved out of the fibres;
c) separating the dissolved collagen-containing fraction from the fibre- and tissue residue;
d) separating the urea from the collagen-containing fraction by means of gradient dialysis so that a collagen-containing aqueous solution is obtained; and
e) renaturing the collagen in phosphate-buffered saline (PBS), a collagen solution with isolated collagen being obtained.

2. Method according to one of the preceding claims, the collagen-containing tissue being isolated rat tail tendons.

3. Method according to one of the preceding claims, the collagen-containing tissue being acellularised pig small intestine.

4. Method according to one of the preceding claims, the urea end concentration being 7 to 12 mol/l in step b).

5. Method according to one of the preceding claims, the urea end concentration being 9 mol/l in step b).

6. Method according to one of the preceding claims, the collagen-containing fibres being agitated with the urea solution in step b).

7. Method according to one of the preceding claims, the collagen-containing fibres being incubated with the urea solution for 12 to 36 hours in step b).

8. Method according to one of the preceding claims, the separation of the dissolved collagen-containing fraction being effected by means of centrifugation and/or filtration in step c).

9. Method according to one of the preceding claims, the separation of the dissolved collagen-containing fraction being effected by fractionation by means of gel filtration and subsequently possibly a plurality of fractions being combined again in step c).

10. Method according to one of the preceding claims, dialysis against water being implemented under cold conditions for 4 to 12 days in step d).

11. Method according to one of the preceding claims, the obtained aqueous collagen solution having a collagen content of 3 to 8 mg/ml.

12. Method according to one of the preceding claims, the obtained aqueous collagen solution being lyophilised in a further step.

13. Method according to one of the preceding claims, the collagen content of the obtained aqueous collagen solution being determined by means of UV spectroscopy (UV).

14. Method according to claim 13, the characteristic amino acid signature of histidine at 213 nm in the UV spectrogram being evaluated for concentration determination.

15. Isolated collagen, producible or produced according to the method according to one of the preceding claims.

16. Collagen according to claim 15, which shows a triple helix signature in circular dichroism spectroscopy (DC).

17. Collagen according to claim 16, the triple helix signature in the DC spectrogram being **characterised by** a negative band between 217 and 227 nm and possibly a weaker negative band in the vicinity of 200 nm.

18. Collagen according to one of the claims 15 to 17, which in UV spectroscopy (UV) shows a characteristic amino acid signature of histidine at 213 nm.

19. Method for the production of a biomatrix, comprising the steps:
implementing steps a) to e) of the method according to one of the claims 1 to 14,
f) mixing the obtained collagen solution with cell culture medium in a ratio of 2 : 1 to 1 : 2 to form a collagen-containing matrix precursor solution; and
g) gelling the matrix precursor solution to form a collagen-containing biomatrix at increased temperature.

20. Biomatrix, producible or produced according to the method according to claim 19.

21. Biomatrix, comprising isolated collagen according to one of the claims 15 to 18.

22. Tissue replacement comprising the biomatrix according to claim 20 or 21 and vital tissue cells which are cultivated in and/or on the biomatrix.

23. Organ replacement, comprising the biomatrix according to claim 20 or 21 and vital tissue cells which are cultivated in and/or on the biomatrix.

24. In vitro test system, comprising the biomatrix according to claim 20 or 21 and vital cells which are cultivated in and/or on the biomatrix.

25. Use of the collagen according to one of the claims 15 to 18 or the biomatrix according to claim 20 or 21 for the production of a tissue replacement.

26. Use of the collagen according to one of the claims 15 to 18 or the biomatrix according to claim 20 or 21 for the production of an organ replacement.

27. Use of the collagen according to one of the claims 15 to 18 or the biomatrix according to claim 20 or 21 for the production of an in vitro test system.

## Revendications

1. Procédé d'isolation de collagène ou de collagène des protéines de la matrice à partir d'un tissu renfermant du collagène comportant les étapes consistant à :
a) isoler les fibres renfermant du collagène du tissu,
b) incuber les fibres renfermant du collagène isolées dans une solution aqueuse contenant de l'urée à une concentration finale de 5 à 10 mol/l, une fraction renfermant du collagène étant séparée des fibres par dissolution,
c) séparation de la fraction renfermant du collagène dissoute du résidu de fibres et de tissu,
d) séparation de l'urée de la fraction renfermant du collagène par dialyse par gradient de façon à obtenir une solution aqueuse renfermant du collagène, et
e) renaturation du collagène dans un sel tamponné au phosphate (PBS), de façon à obtenir une solution de collagène avec le collagène isolé.

2. Procédé conforme à la revendication 1,
sur lequel le tissu renfermant du collagène est un tendon de queue de rat.

3. Procédé conforme à l'une des revendications précédentes selon lequel le tissu renfermant du collagène est de l'intestin grêle de porc acellulaire.

4. Procédé conforme à l'une des revendications précédentes selon lequel dans l'étape b) la concentration finale en urée s'élève à 7 à 12 mol/l.

5. Procédé conforme à l'une des revendications précédentes selon lequel dans l'étape b) la concentration finale en urée s'élève à 9 mol/l.

6. Procédé conforme à l'une des revendications précédentes selon lequel dans l'étape b) les fibres renfermant du collagène sont agitées avec la solution d'urée.

7. Procédé selon l'une des revendications précédentes selon lequel dans l'étape b) les fibres renfermant du collagène sont incubées avec la solution d'urée pendant 12 à 36 heures.

8. Procédé conforme à l'une des revendications précédentes selon lequel dans l'étape c) la séparation de la fraction renfermant du collagène dissout est effectuée par centrifugation et/ ou filtration.

9. Procédé conforme à l'une des revendications précédentes selon lequel dans l'étape c) la séparation de la fraction renfermant du collagène dissout est effectuée par fractionnement par filtration sur gel puis, le cas échéant plusieurs fractions sont à nouveau rassemblées.

10. Procédé conforme à l'une des revendications précédentes selon lequel dans l'étape d), on effectue une dialyse par circulation d'eau à contre courant à basse température pendant 4 à 12 jours.

11. Procédé conforme à l'une des revendications précédentes selon lequel la solution aqueuse de collagène obtenue présente une teneur en collagène de 3 à 8 mg/ml.

12. Procédé conforme à l'une des revendications précédentes selon lequel dans une étape ultérieure, on lyophilise la solution de collagène obtenue.

13. Procédé conforme à l'une des revendications précédentes selon lequel la teneur en collagène de la solution aqueuse de collagène obtenue est déterminée par spectroscopie UV.

14. Procédé conforme à la revendication 13 selon lequel pour déterminer la concentration dans le spectrogramme UV, on évalue la signature d'acides aminés caractéristique de l'histidine à 213 nm.

15. Collagène isolé pouvant être obtenu ou obtenu par la mise en oeuvre du procédé conforme à l'une des revendications précédentes.

16. Collagène conforme à la revendication 15 dans lequel la spectroscopie par dichroïsme circulaire (DC) montre une signature de triple hélice.

17. Collagène conforme à la revendication 16 dans lequel la signature de triple hélice dans le spectrogramme DC est **caractérisée par** une bande négative entre 217 et 227 nm et le cas échéant une bande négative plus faible à proximité de 200 nm.

18. Collagène conforme à l'une des revendications 15 à 17 présentant en spectroscopie UV une signature d'acide aminés caractéristique de l'histidine à 213 nm.

19. Procédé d'obtention d'une biomatrice comportant les étapes consistant à :
mettre en oeuvre les étapes a) à e) du procédé conforme à l'une des revendications 1 à 14,
f) mélanger la solution de collagène obtenue avec un milieu de culture cellulaire dans un rapport de 2/1 à 1/2 pour obtenir une solution précurseur de matrice renfermant du collagène, et
g) gélifier cette solution précurseur de matrice pour obtenir une biomatrice renfermant du collagène à température élevée.

20. Biomatrice pouvant être obtenue ou obtenue par la mise en oeuvre du procédé conforme à la revendication 19.

21. Biomatrice renfermant du collagène isolé conforme à l'une des revendications 15 à 18.

22. Substitut de tissu renfermant une biomatrice conforme à la revendication 20 ou à la revendication 21 et des cellules de tissu vitales qui sont cultivées dans et/ou sur la biomatrice.

23. Substitut d'organe renfermant la biomatrice conforme à la revendication 20 ou à la revendication 21 et des cellules de tissu vitales qui sont cultivées dans et/ou sur la biomatrice.

24. Système de test in vitro renfermant la biomatrice conforme à la revendication 20 ou à la revendication 21 et des cellules vitales qui sont cultivées dans et/ou sur la biomatrice.

25. Utilisation du collagène conforme à l'une des revendications 15 à 18 ou de la biomatrice conforme à la revendication 20 ou à la revendication 21 pour l'obtention d'un substitut de tissu.

26. Utilisation du collagène conforme à l'une des revendications 15 à 18 ou de la biomatrice conforme à la revendication 20 ou à la revendication 21 pour l'obtention d'un substitut d'organe.

27. Utilisation du collagène conforme à l'une des revendications 15 à 18 ou de la biomatrice conforme à la revendication 20 ou à la revendication 21 pour l'obtention d'un système de test in vitro.
